# EUROPEAN PATENT SPECIFICATION

(11) **EP 3 669 896 B1**
(45) Date of publication and mention of the grant of the patent: **28.02.2024**
(21) Application number: 19215647.9
(22) Date of filing: 12.12.2019
(51) Int. Cl.: A61L 2/20

(54) **GENERATOR OF HYDROGEN PEROXIDE IN VAPOUR PHASE**
WASSERSTOFFPEROXID-VERDAMPFER
VAPORISATEUR DE PEROXYDE D'HYDROGÈNE

(30) Priority: 17.12.2018 IT 201800011162
(43) Date of publication of application: 24.06.2020
(73) Proprietor: Amira S.r.l., 20844 Truiggio (Monza Brianza) (IT)
(72) Inventor: DELMIGLIO, Angelo, 20844 TRIUGGIO (MONZA BRIANZA) (IT); DELMIGLIO, Mattia, 20844 TRIUGGIO (MONZA BRIANZA) (IT)
(74) Representative: Inchingalo, Simona

(56) References cited:
- EP-A1- 1 283 061
- EP-A1- 1 413 317
- EP-A2- 1 839 679
- WO-A1-2006/101467
- WO-A1-2015/175564

## Description

The present invention relates to a sterilisation apparatus and a sterilisation method.

In particular, the sterilisation apparatus according to the present invention uses vaporized hydrogen peroxide to carry out the sterilisation process.

In fact, the exposure to hydrogen peroxide allows the sterilisation of objects and spaces with a high degree of effectiveness.

Furthermore, such sterilisation method, also called bio-decontamination, does not release toxic agents on the treated materials, making its use particularly advantageous.

The present invention can therefore be applied in all areas where there is a need for biological decontamination. Among the various applications we can mention, for example, the sterilisation of rooms, pass boxes, autoclaves, containers, pressurized and vacuum volumes.

Nowadays there are a multitude of machines on the market which allow the sterilisation of spaces and objects by exploiting the vaporized hydrogen peroxide.

Such machines have a vaporizer inside which the hydrogen peroxide is vaporized following a sequence of thermodynamic transformations which are predetermined and pre-set on a control unit that manages its operation.

The vaporized hydrogen peroxide is then placed inside a work volume to be sterilized or wherein the objects to be sterilized have previously been placed.

Disadvantageously, the process parameters which define the thermodynamic transformations, especially in the less recent machines, must be set and changed by a specialized operator, resulting in an increase in operating costs and leaving part of the accuracy of the vaporization process to an operator.

Such process is of fundamental importance and must be performed according to a predetermined succession of thermodynamic states in order to obtain hydrogen peroxide in the gas phase able to ensure a highly effective sterilisation.

Some machines have automatic controllers which, by evaluating some predetermined parameters by means of appropriate temperature sensors, are able to independently define the process parameters for an effective performance of the aforementioned thermodynamic transformations. Such machines are configured to perform sterilisation processes in work volumes at atmospheric pressure or in conditions of slight depression or slight overpressure.

However, very disadvantageously, the machines of the prior art are not able to ensure an effective sterilisation of work volumes maintained in conditions of low operating pressures, or of low or medium vacuum, for example, in the order of 15 absolute mbar (1,5 KPa).

In such conditions, in fact, the machine tends to suck in the contaminants present in the surrounding environment due to the high pressure difference.

In this way the work volume is constantly contaminated by the external environment and, therefore, the effectiveness of the sterilisation process is compromised.

Disadvantageously, moreover, the prior art machines have particularly high production and maintenance costs which often tend to limit their use. WO 2006/101467, WO 2015/175564 and EP 1839679 disclose respective devices for producing vapour of hydrogen peroxide; EP 1283061 and EP 1413317 disclose respective devices using such vapour.

In this context, the aim of the present invention is to propose a sterilisation apparatus and a sterilisation method which overcome at least some of the drawbacks of the prior art mentioned above.

A first technical task underlying the present invention is to provide a sterilisation apparatus and a sterilisation method which ensure a high versatility and a remarkable adaptability to a plurality of different operating conditions of the work volume.

A second aim of the present invention is to increase the effectiveness of the sterilisation process by limiting the possibility of contamination with respect to the known sterilisation apparatuses.

A further technical task is to ensure an effective sterilisation within work volumes in conditions of low pressures, for example in the order of 15 absolute mbar.

In particular, another aim of the present invention is to provide a sterilisation apparatus which is able of sterilizing work volumes both at atmospheric pressure and at pressures in the order of about 2.5 absolute bar, and in the aforementioned conditions of low pressure.

The mentioned technical aim and the specified tasks are substantially achieved by a sterilisation apparatus and a sterilisation method comprising the technical specifications set out in one or more of the appended claims. Further characteristics and advantages of the present invention will become more apparent from the description of an exemplary, but not exclusive, and therefore non-limiting preferred embodiment of a sterilisation apparatus and a sterilisation method, as illustrated in the appended figures, wherein:

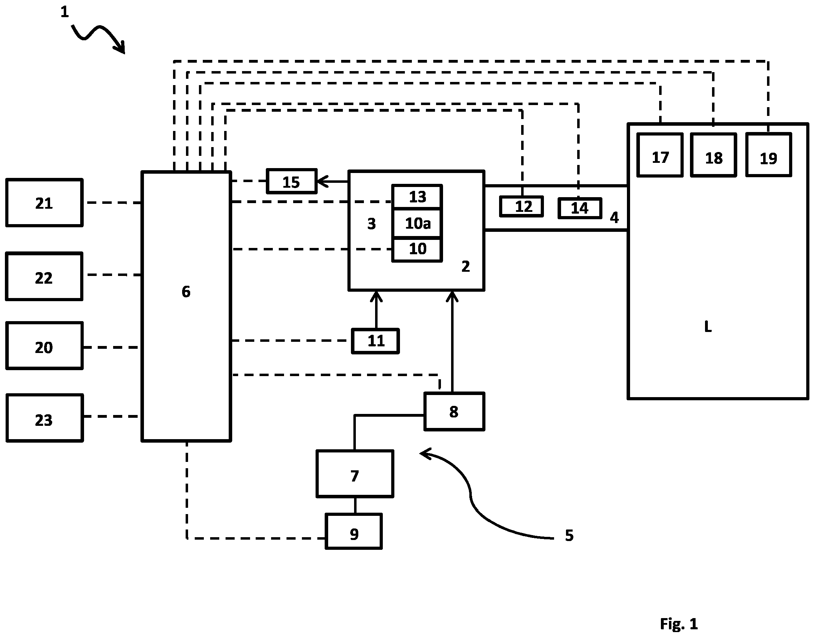

- figure 1 shows a schematic view of a sterilisation apparatus according to a possible embodiment of the present invention;
- figure 2 shows a schematic view of a sterilisation apparatus according to a possible further embodiment of the present invention;

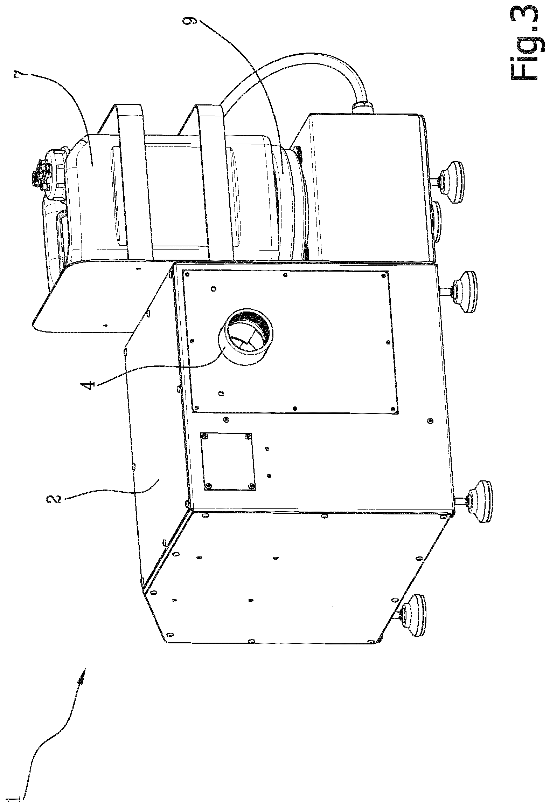

- figure 3 shows a perspective view of the sterilisation apparatus according to the present invention.

With reference to the attached figures, a sterilisation apparatus according to the present invention has been indicated as a whole with the reference number 1.

The sterilisation apparatus 1 according to the present invention is a vaporized hydrogen peroxide sterilisation apparatus.

In the following discussion, reference is made to the efficient and instantaneous vaporization of the hydrogen peroxide. Efficient vaporization means a change of state from liquid to vapour which maximizes the amount of hydrogen peroxide which is obtained in the vapour state, minimizing or preferably eliminating the degradation of hydrogen peroxide into water and oxygen. Instant vaporization means a sudden change of state from liquid to vapour. The double need to have a vaporization which is at the same time efficient and instantaneous, requires that the change of state occurs within a well-defined temperature range. An excessively high temperature ensures instantaneous but not efficient vaporization, while an excessively low temperature leads to a slow and inefficient vaporization. The correct temperature range depends on the pressure of the environment in which the change of state occurs.

In particular, the sterilisation apparatus 1 is configured to introduce a quantity of vaporized hydrogen peroxide into a work volume "L" to obtain the sterilisation of a space and/or objects contained therein.

The sterilisation apparatus 1 comprises a vaporizer 2 defining a containment volume 3, inside which a quantity of liquid hydrogen peroxide is introduced, and an output duct 4 which allows the outflow of the hydrogen peroxide in gas phase towards the work volume "L".

In other words, the sterilisation apparatus 1 comprises a V-PHP generator *(Vapor-Phase Hydrogen Peroxide).*

Preferably, the liquid hydrogen peroxide is fed in the form of an aqueous solution with a concentration of 35% of liquid hydrogen peroxide. Advantageously, the containment volume 3 is of the vacuum tight type so as to prevent external contaminants from entering the containment volume 3 contaminating it and affecting the effectiveness of the sterilisation process. Preferably, the vacuum tight containment volume 3 is also of the pressure tight type.

Preferably, with a vacuum tight containment volume it is meant that the containment volume 3 is able to undergo a pressure loss of less than 1.3 mbar per minute for 10 minutes after having stabilized its internal pressure at 70 absolute mbar. Advantageously, the same tight containment volume is also able to undergo a pressure loss of less than 30 mbar in 10 minutes once the internal pressure reaches and is stabilized at 1.5 relative bar (2500 absolute mbar).

In this way, thanks to the outlet duct 4, in full operation, the pressure inside the containment volume 3 is substantially equal to that inside the work volume "L".

In particular, the liquid hydrogen peroxide is introduced into the containment volume 3 of the vaporizer 2 by suitable means 5 for feeding hydrogen peroxide in liquid phase.

Advantageously, the means 5 for feeding peroxide in liquid phase are connected to a control unit 6 configured to regulate the feeding of peroxide in liquid phase to the containment volume 3.

In other words, the sterilisation apparatus 1 comprises a control unit 6 which controls the means 5 for feeding liquid hydrogen peroxide to the containment volume 3.

Preferably, the means 5 for feeding liquid hydrogen peroxide comprise a reservoir 7 adapted to contain a quantity of hydrogen peroxide in the liquid state and a pump 8 configured for moving the hydrogen peroxide from the reservoir 7 to the vaporizer 2.

Advantageously, the aforementioned pump 8 can be a peristaltic pump in order to limit the risk of contamination of the liquid hydrogen peroxide by agents external to the sterilisation apparatus 1.

Particularly, the sterilisation apparatus 1 can comprise a dynamometer (or a weighing scale) 9 connected to the reservoir 7 to obtain, instant by instant, a measurement of the quantity of hydrogen peroxide contained in the reservoir.

According to a possible embodiment of the present invention, the dynamometer 9 can also be connected to the control unit 6 to send the measurement obtained to the aforementioned control unit 6.

In this way, the dynamometer 9 provides the control unit 6 with a measurement which indicates the amount of hydrogen peroxide consumed during the process.

Therefore, the dynamometer 9 ensures and allows to monitor in real time the compliance of the mass flow of liquid hydrogen peroxide with respect to the pre-set theoretical parameters.

The vaporizer 2 is configured to induce a phase change on the hydrogen peroxide from the liquid phase to the gas phase, so that it can be introduced into the work volume "L".

In other words, the vaporizer 2 induces on the liquid hydrogen peroxide a plurality of thermodynamic transformations which determine a phase change of the hydrogen peroxide bringing it to the gas phase.

Such thermodynamic transformations must occur according to predetermined temperature and pressure conditions to maximize the efficiency of the process.

The aforementioned temperature and pressure conditions are reached in the containment volume 3 thanks to the presence of at least one heating element 10.

Preferably, moreover, the vaporizer 2 can comprise suitable means to create a vapour flow from the containment volume to the work volume.

In some embodiments, the vaporizer 2 can comprise a heating element 10 and insufflation means 11, which will be described later in greater detail, suitable to define the conditions of temperature and pressure appropriate for carrying out the thermodynamic transformations.

In particular, the heating element 10 is configured to provide the liquid hydrogen peroxide with the amount of heat necessary to carry out the aforementioned change of state.

According to a possible embodiment, the heating element 10 comprises a heat exchange surface 10a (or evaporation surface) and one or more devices (typically resistors) suitable to transmit heat to the heat exchange surface 10a. The heat exchange surface 10a is suitable to receive the flow of liquid hydrogen peroxide and to promote the change to the vapour phase.

According to possible embodiment of the present invention, the heat exchange surface 10a is suitable to receive a flow of liquid hydrogen peroxide to promote its instantaneous change of state.

Preferably, the heating element 10 is made of metallic material, even more preferably a metallic material with a high thermal conductivity. Advantageously, the heating element 10 can be made of aluminum so as to ensure an efficient heat transmission and a fast response to the thermal variations imposed, for example, by the control unit 6.

According to further possible embodiments, the heating element 10 can have a different conformation without altering the inventive concept underlying the present invention.

Furthermore, in some embodiments, heating means 12 can be provided arranged in the outlet duct 4 and configured to provide a predetermined amount of heat to the hydrogen peroxide in the gas phase, so that it has optimal characteristics for sterilisation upon entering the work volume "L". In other words, the outlet duct 4 can comprise heating means 12 suitable to modify the temperature of the hydrogen peroxide in the gas phase during the outflow from the vaporizer 2.

The possible insufflation means 11 are configured to define the predetermined pressure conditions within the containment volume 3 and are in any case suitable to create a vapour flow from the containment volume to the work volume.

Particularly the sterilisation apparatus 1 can comprise insufflation means 11 active on the vaporizer 2 for introducing a gas flow inside the containment volume 3.

According to possible embodiment of the present invention, the insufflation means 11 can comprise a connection with a compressed air flow pipe, preferably pre-filtered by an external plant.

According to another embodiment of the present invention, the insufflation means 11 can comprise a blower suitable for sucking air from the external environment, filtering it and introducing it into the containment volume 3. According to a further embodiment, the insufflation means 11 can comprise filtering elements for the air outside the sterilisation apparatus 1, without altering the inventive concept underlying the present invention.

Such operating solution is particularly advantageous for carrying out the sterilisation process in conditions in which the work volume "L" has a pressure lower than the external pressure, for example of 15 absolute mbar.

In fact, the air flow from the outside to the inside of the sterilisation apparatus 1 can be promoted by the pressure difference established between the two environments. In other words, the pressure difference sucks the air inside the vaporizer.

The vaporizer 2 comprises at least one temperature sensor 13 configured to detect a temperature of the heating element 10, preferably to detect a temperature of the heat exchange surface 10a.

Advantageously, the presence of the temperature sensors 13 on the heating element ensures a high accuracy measurement of the temperature on the heat exchange surface 10a.

In particular, the temperature sensor 13 generates a temperature signal representing a temperature of the heating element 10 and sends such temperature signal to the control unit 6.

In more detail, the temperature sensor 13 generates a temperature signal representing the temperature of the heat exchange surface 10a of the heating element 10.

Advantageously, such temperature signal allows the control unit 6 to control the temperature on the heat exchange surface 10a in order to ensure the efficient and instantaneous vaporization of the hydrogen peroxide.

In this way, the sterilisation apparatus 1 ensures a highly efficient vaporization which maximizes the concentration of hydrogen peroxide in the gas state, for example, measured in PPM (parts per million). Particularly, the efficient vaporization preserves the hydrogen peroxide molecule, determining its change of state (from liquid to gaseous), limiting, preferably preventing, the formation of waste products such as water and/or oxygen thanks to the temperature feedback control of the heating element 10.

Preferably, the pump 8 is controlled by the control unit 6 in order to maintain a constant inflow of hydrogen peroxide on the heat exchange surface 10a of the heating element 10 so as to ensure the continuity of constant heat subtraction over time.

In this way the control unit 6, controlling the pump 8 of the liquid hydrogen peroxide and the temperature of the heating element 10, allows the efficient and instantaneous vaporization of the hydrogen peroxide. Particularly, the control unit 6 maintains the temperature of the heat exchange surface 10a constant as the flow rate of the liquid hydrogen peroxide varies.

In other words, the control unit 6, on the base of a preloaded logic, maintains the temperature of the heat exchange surface 10a in balance by feeding the heating element and subtracting heat through the flow of hydrogen peroxide.

Advantageously, also the signal supplied by the dynamometer 9 allows the control unit 6 to control the pump 8 according to a feedback logic so as to ensure the correct mass flow of hydrogen peroxide to the vaporizer 2. By way of example, it is possible that in the reservoir 7 and/or in the duct which connects it to the pump 8 a gas bubble is formed, typically air, water vapour or oxygen deriving from the natural degradation of hydrogen peroxide. In this case the volume flow rate delivered by the pump 8 is correct, but the actual mass flow of hydrogen peroxide deviates from the desired value. In this case, to avoid that in the long run the real value of the mass of hydrogen peroxide delivered deviates considerably from its theoretical value, the signal supplied by the dynamometer 9 allows the control unit 6 to control the pump 8 to carry out small flow rate corrections. The corrections are preferably small enough not to significantly affect the amount of heat instantly removed by the hydrogen peroxide from the heat exchange surface 10a.

Moreover, the measurements of the dynamometer 9 provide a certain datum related to the mass of hydrogen peroxide actually delivered, so as to ensure that the actual sterilisation process is in accordance with the theoretical sterilisation process.

According to a possible embodiment and as illustrated in the attached figures, the output duct 4 comprises a second temperature sensor 14 adapted to generate a second temperature signal representing the temperature of the peroxide in the gas phase in the outlet duct 4 or of the temperature of the output duct 4.

Advantageously, also the second temperature sensor 14 sends the second temperature signal to the control unit 6.

Particularly, the control unit 6 controls the heating means 12 according to the second temperature signal so as to keep the hydrogen peroxide in the gas state and limiting, preferably preventing, condensation phenomena during the passage inside the output duct 4 connecting the containment volume 3 to the work volume "L".

The sterilisation apparatus 1 further comprises at least one pressure sensor 15 adapted to generate a pressure signal representing the pressure within the containment volume 3 and/or the work volume "L". Particularly, the pressure sensor 15 is configured to send such pressure signal to the control unit 6.

Preferably, the control unit 6 modifies the temperature on the heat exchange surface 10a according to the pressure signal sent by the pressure sensor 15 located inside the containment volume 3 and/or the work volume "L".

Preferably, the sterilisation apparatus 1 further comprises one or more of a relative humidity sensor 18, a third temperature sensor 17, and a peroxide concentration sensor 19.

Each of such sensors is suitable for generating a signal representing its physical quantity (temperature, relative humidity and peroxide concentration) within the work volume "L", and sending such signal to the control unit 6.

Advantageously, the heating element 10 and the feeding means 5 are operatively connected to the control unit 6 which regulates their operation on the basis of the signals sent by the temperature sensor 13 and by the pressure sensor 15 and on the basis of a predefined logic.

In other words, the control unit 6 controls the operation of the heating element 10, of the feeding means 5 of the liquid hydrogen peroxide and/or of the possible heating means 12, on the basis of the signals sent by the aforementioned plurality of sensors and on basis of a predefined logic. Particularly, the control unit 6 receives and processes a plurality of signals generated by the sensors of the sterilisation apparatus 1 and controls the heating element and the feeding means 5 of the liquid hydrogen peroxide to carry out the vaporization on the basis of a predefined logic.

Preferably, the control unit 6 is also suitable to control ventilation means 16 configured to remove/inject a fluid (typically air or a mixture of air and gaseous hydrogen peroxide) from/in the working volume "L" on the basis of a preloaded logic.

In other words, the control unit 6 can be configured to control ventilation means 16 which allow to bring and maintain the work volume "L" in a predetermined pressure condition.

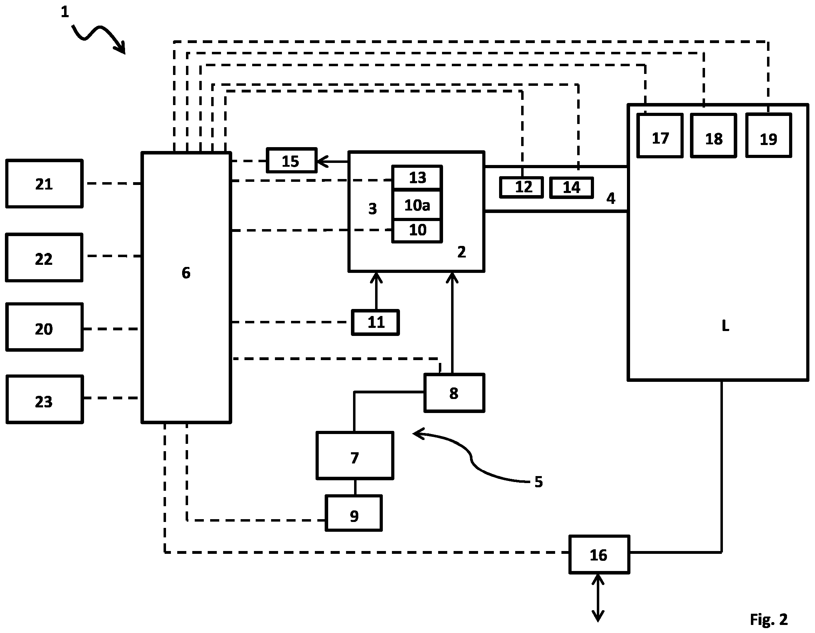

According to a possible embodiment and as illustrated in figure 2, the sterilisation apparatus 1 comprises the aforementioned ventilation means 16.

Particularly, the ventilation means 16 can comprise a catalyst filter which prevents the hydrogen peroxide sucked from the work volume "L" from damaging its components.

Preferably, the control unit 6 is connectable to at least one external peripheral unit for sending and/or receiving a plurality of operational parameters.

Particularly, the control unit 6 can be connected to an external computer 23, to a Programmable Logic Controller (or PLC), to a tablet, to a server 22, to a printer 21 and/or to a monitor. The external peripheral unit, for example, can be suitable to enter operational parameters or simply to allow viewing and/or archiving of at least some parameters related to the sterilisation process, for example the measurements collected by the pressure and temperature sensors, by the dynamometer, etc.

Moreover, the control unit 6 can comprise a user interface module 20 configured to allow the insertion of a plurality of instructions related to the operation of the sterilisation apparatus 1 and/or to display operational parameters during operation.

According to a further aspect, the present invention relates to a sterilisation method comprising the steps of providing a work volume and a step of providing a vaporizer defining a vacuum tight containment volume and comprising a heating element.

Preferably the method can be implemented with a sterilisation apparatus 1 according to the invention, as described above.

The method further comprises a step of providing a pressure sensor to detect at least one measurement related to the pressure inside the containment volume or inside the work volume.

Moreover, the method provides for a step of detecting by means of the pressure sensor at least one measurement related to the pressure inside the containment volume or inside the work volume.

The method may further comprise a step of providing a temperature sensor to detect at least one measurement related to the temperature of the heating element.

Moreover, the method provides for a step of detecting by the temperature sensor at least one measurement related to the temperature of the heating element, preferably of a heat exchange surface 10a of the heating element.

The method can also provide a step of sucking/inject a quantity of a fluid from/into the work volume.

Advantageously, such step allows to set and maintain the work volume at a predetermined pressure.

According to an operating configuration, for example, the aforementioned suction phase can remove a quantity of fluid from the work volume in order to carry out the sterilisation process at a pressure of 15 absolute mbar. Preferably, such suction step comprises a step of filtering the fluid sucked from the work volume by means of a catalyst.

The method comprises a step of feeding an amount of liquid hydrogen peroxide on the heating element.

In other words, a quantity of peroxide is placed inside the vaporizer near the heating element.

Preferably, the step of feeding a quantity of liquid hydrogen peroxide provides a sub-step of feeding the liquid hydrogen peroxide in the form of a flow to the heat exchange surface of the heating element.

Preferably, the method comprises a step of ventilating the containment volume by means of appropriate insufflation means.

The method further provides for a step of operating the heating element for inducing a phase change in the hydrogen peroxide from the liquid phase to the gas phase.

Particularly, such step of feeding a quantity of liquid hydrogen peroxide can occur during the step of operating the heating element.

The method further comprises a step of introducing the hydrogen peroxide in gas phase in the work volume.

Preferably, the method may comprise a step of providing heating means in a duct which inject hydrogen peroxide into the work volume. Preferably the method also comprises the step of operating the heating means to obtain hydrogen peroxide in the gas phase at a predetermined temperature. Particularly, the step of feeding the liquid hydrogen peroxide and the step of operating the heating element are controlled on the base of a predefined logic and on the base of the measurement related to the pressure inside the containment volume and/or inside the work volume and on the base of the measurement related to the temperature of the heating element.

In other words, the heating element can be controlled with a retroactive logic, for example, based on the temperature measurement of the heat exchange surface, in order to guarantee an efficient and instantaneous vaporization of the hydrogen peroxide.

Furthermore, the method can comprise the step of providing a dynamometer (or a weighing scale) and the step of detecting by the dynamometer a signal relating to the mass flow of the hydrogen peroxide. Advantageously, the method can also comprise a step of comparing the signal related to the actual mass flow of the hydrogen peroxide with the value of the theoretical mass flow of the hydrogen peroxide.

Particularly, the method allows the pump to be controlled according to a feedback logic by ensuring a mass flow of hydrogen peroxide to the vaporizer so as to allow an efficient and instantaneous vaporization.

In this way, the method allows to minimize the difference between the actual mass flow of the hydrogen peroxide and the theoretical mass flow value of the hydrogen peroxide.

It is therefore observed that the present invention achieves the proposed aim thanks to a sterilisation apparatus and a sterilisation method which ensure a high versatility and a remarkable adaptability to a multiplicity of different operating conditions of the work volume thanks to the presence of a vacuum tight containment volume which allows the sterilisation process to be carried out at different pressure conditions, preventing the contamination of the work volume by external contaminants. Advantageously, the sterilisation apparatus and the sterilization method according to the present invention ensure an effective sterilisation within work volumes in conditions of low pressures, for example in the order of 15 mbar.

Particularly, the present invention makes it possible to sterilize work volumes both at environmental pressures and in the aforementioned pressure conditions.

Furthermore, the sterilisation apparatus and the sterilisation method according to the present invention increase the effectiveness of the sterilisation process by limiting the possibility of contamination with respect to known sterilisation apparatuses.

## Claims

1. Sterilisation apparatus (1) comprising:
- means (5) for feeding hydrogen peroxide in liquid phase;
- a vaporizer (2) suitable for inducing a phase change for the hydrogen peroxide from liquid phase to gas phase, and defining a vacuum tight containment volume (3) comprising:
a temperature sensor (13),
a heating element (10), and
an output duct (4) for the outflow of the hydrogen peroxide in gas phase toward a work volume (L);
- a pressure sensor (15),
- a control unit (6)
wherein
the pressure sensor (15) is operatively connected to the control unit (6), the pressure sensor (15) being adapted to generate a pressure signal representing the pressure inside the containment volume (3) or inside the work volume (L) and to send the pressure signal to the control unit (6);
the temperature sensor (13) is operatively connected to the control unit; the means (5) for feeding hydrogen peroxide are operatively connected to the control unit (6);
the heating element (10) is operatively connected to the control unit (6);
and wherein the control unit (6) controls the operation of the heating element and of the means (5) for feeding hydrogen peroxide on the base of the signals sent by the temperature sensor (13) and by the pressure sensor (15) and on the base of a predefined logic;
**characterized in that** the temperature sensor (13) is adapted to generate a temperature signal representing a temperature of the heating element (10) and to send the temperature signal to the control unit (6).

2. Sterilisation apparatus according to claim 1, wherein said heating element (10) defines a heat exchange surface (10a), said heating element (10) being adapted to receive a predetermined quantity of hydrogen peroxide; wherein said temperature sensor (13) is configured to generate at least one temperature signal representative of a temperature on said heat exchange surface (10a) and to send the temperature signal to the control unit (6).

3. Sterilisation apparatus according to claim 1 or 2, wherein the output duct (4) comprises heating means (12) configured for providing a predetermined amount of heat to the hydrogen peroxide in gas phase.

4. Sterilisation apparatus according to claim 3, wherein the output duct (4) comprises a second temperature sensor (14) adapted to generate a second temperature signal representing the temperature of the output duct (4) and to send the second temperature signal to the control unit (6); wherein the heating means (12) are operatively connected to the control unit (6); and wherein the control unit (6) controls the operation of the heating means (12) on the base of the signals sent by the second temperature sensor (14) and on the base of a predefined logic.

5. Sterilisation apparatus according to one or more of the preceding claims, further comprising one or more among:
- a third temperature sensor (17);
- a relative humidity sensor (18);
- a peroxide concentration sensor (19).

6. Sterilisation apparatus according to one or more of the preceding claims, wherein the control unit (6) is adapted to control ventilation means (16) configured for removing a fluid from the work volume (L) on the base of a preloaded logic.

7. Sterilisation apparatus according to preceding claim, further comprising the ventilation means (16).

8. Sterilisation apparatus according to one or more of the preceding claims, wherein the means (5) for feeding hydrogen peroxide comprise a reservoir (7), adapted to contain a quantity of hydrogen peroxide, and a pump (8) configured for moving the hydrogen peroxide from the reservoir (7) to the vaporizer (2).

9. Sterilisation apparatus according to claim 8, further comprising a dynamometer (9) connected to said reservoir (7) for obtaining a measurement of the quantity of hydrogen peroxide contained in the reservoir (7) and configured for sending the measurement to the control unit (6).

10. Sterilisation apparatus according to one or more of the preceding claims, comprising blowing means (11) active on the vaporizer (2) for introducing a flow of gas or of gas mixtures inside the containment volume (3).

11. Sterilisation apparatus according to one or more of the preceding claims, wherein the control unit (6) is suitable for being connected to at least one external peripheral unit for sending and/or receiving a plurality of operational parameters.

12. Sterilisation method comprising the steps of:
- providing a work volume
- providing a vaporizer defining a vacuum tight containment volume and comprising a heating element;
- providing a pressure sensor;
- providing a temperature sensor;
- detecting by means of the pressure sensor at least one measurement related to the pressure inside the containment volume or inside the work volume;
- detecting by the temperature sensor at least one measurement related to the temperature of the heating element;
- feeding an amount of liquid hydrogen peroxide on the heating element;
- operating the heating element for inducing a phase change in the hydrogen peroxide from the liquid phase to the gas phase;
- introducing the hydrogen peroxide in gas phase in the work volume; wherein the step of feeding the liquid hydrogen peroxide and the step of operating the heating element are controlled on the base of a predefined logic and on the base of the measurement related to the pressure inside the containment volume or inside the work volume and on the base of the measurement related to the temperature of the heating element.

13. Method according to the previous claim, wherein the step of detecting by means of the temperature sensor at least one measurement related to the temperature of the heating element comprises the sub-step of detecting at least one measurement related to the temperature of the heat exchange surface of the heating element.

14. Method according to claim 12 or 13, further comprising the steps of:
- providing a dynamometer (9);
- detecting by the dynamometer (9) to detect a signal related to the actual mass flow of the hydrogen peroxide;
- compare the signal related to the actual mass flow of the hydrogen peroxide with the value of the theoretical mass flow of the hydrogen peroxide;
- command the pump (8) so as to minimize the difference between the actual mass flow of the hydrogen peroxide and the theoretical mass flow value of the hydrogen peroxide.

## Patentansprüche

1. Sterilisationsvorrichtung (1), umfassend:
- Mittel (5) zum Zuführen von Wasserstoffperoxid in flüssiger Phase;
- einen Verdampfer (2), der geeignet ist, einen Phasenwechsel für das Wasserstoffperoxid von der flüssigen Phase in die gasförmige Phase einzuleiten und ein vakuumdichtes Auffangvolumen (3) zu definieren, umfassend:
einen Temperatursensor (13),
ein Heizelement (10), und
eine Ausgangsleitung (4) für das Ausströmen des Wasserstoffperoxids in der gasförmigen Phase in Richtung eines Nutzvolumens (L);
- einen Drucksensor (15),
- eine Steuereinheit (6),
wobei
der Drucksensor (15) betriebswirksam mit der Steuereinheit (6) verbunden ist, wobei der Drucksensor (15) ausgebildet ist, ein Drucksignal zu erzeugen, das den Druck im Inneren des Auffangvolumens (3) oder im Inneren des Nutzvolumens (L) darstellt, und das Drucksignal an die Steuereinheit (6) zu senden;
der Temperatursensor (13) betriebswirksam mit der Steuereinheit verbunden ist;
die Mittel (5) zum Zuführen von Wasserstoffperoxid betriebswirksam mit der Steuereinheit (6) verbunden sind;
das Heizelement (10) betriebswirksam mit der Steuereinheit (6) verbunden ist;
und wobei die Steuereinheit (6) den Betrieb des Heizelements und der Mittel (5) zum Zuführen von Wasserstoffperoxid auf der Grundlage der Signale, die vom Temperatursensor (13) und vom Drucksensor (15) gesendet werden, und auf der Grundlage einer vorgegebenen Logik steuert;
**dadurch gekennzeichnet, dass** der Temperatursensor (13) ausgebildet ist, ein Temperatursignal zu erzeugen, das eine Temperatur des Heizelements (10) darstellt, und das Temperatursignal an die Steuereinheit (6) zu senden.

2. Sterilisationsvorrichtung nach Anspruch 1, wobei das Heizelement (10) eine Wärmetauschfläche (10a) definiert, wobei das Heizelement (10) ausgebildet ist, um eine vorgegebene Menge Wasserstoffperoxid aufzunehmen; wobei der Temperatursensor (13) ausgelegt ist, um mindestens ein Temperatursignal zu erzeugen, das für eine Temperatur auf der Wärmeaustauschfläche (10a) repräsentativ ist, und das Temperatursignal an die Steuereinheit (6) zu senden.

3. Sterilisationsvorrichtung nach Anspruch 1 oder 2, wobei die Ausgangsleitung (4) Heizmittel (12) umfasst, die ausgelegt sind, um dem Wasserstoffperoxid in der gasförmigen Phase eine vorgegebene Wärmemenge zuzuführen.

4. Sterilisationsvorrichtung nach Anspruch 3, wobei die Ausgangsleitung (4) einen zweiten Temperatursensor (14) umfasst, der ausgebildet ist, ein zweites Temperatursignal zu erzeugen, das die Temperatur der Ausgangsleitung (4) darstellt, und das zweite Temperatursignal an die Steuereinheit (6) zu senden; wobei die Heizmittel (12) betriebswirksam mit der Steuereinheit (6) verbunden sind; und wobei die Steuereinheit (6) den Betrieb der Heizmittel (12) auf der Grundlage der Signale, die vom zweiten Temperatursensor (14) gesendet werden, und auf der Grundlage einer vorgegebenen Logik steuert.

5. Sterilisationsvorrichtung nach einem oder mehreren der vorhergehenden Ansprüche, ferner umfassend einen oder mehrere unter:
- einem dritten Temperatursensor (17);
- einem Sensor für die relative Luftfeuchtigkeit (18);
- einem Sensor zur Bestimmung der Wasserstoffperoxidkonzentration (19).

6. Sterilisationsvorrichtung nach einem oder mehreren der vorhergehenden Ansprüche, wobei die Steuereinheit (6) ausgebildet ist, Belüftungsmittel (16) zu steuern, die ausgelegt sind, um ein Fluid aus dem Nutzvolumen (L) auf der Grundlage einer vorinstallierten Logik zu entfernen.

7. Sterilisationsvorrichtung nach dem vorhergehenden Anspruch, ferner umfassend die Belüftungsmittel (16).

8. Sterilisationsvorrichtung nach einem oder mehreren der vorhergehenden Ansprüche, wobei die Mittel (5) zum Zuführen von Wasserstoffperoxid einen Behälter (7), der ausgebildet ist, eine Menge Wasserstoffperoxid zu enthalten, und eine Pumpe (8) umfassen, die ausgelegt ist, um das Wasserstoffperoxid vom Behälter (7) zum Verdampfer (2) zu bewegen.

9. Sterilisationsvorrichtung nach Anspruch 8, ferner umfassend ein Dynamometer (9), das mit dem Behälter (7) verbunden ist, um eine Messung der Menge Wasserstoffperoxid, die in dem Behälter (7) enthalten ist, zu erhalten, und das ausgelegt ist, um die Messung an die Steuereinheit (6) zu senden.

10. Sterilisationsvorrichtung nach einem oder mehreren der vorhergehenden Ansprüche, umfassend Blasmittel (11), die auf den Verdampfer (2) wirken, um einen Strom aus Gas oder Gasgemischen in das Auffangvolumen (3) einzuleiten.

11. Sterilisationsvorrichtung nach einem oder mehreren der vorhergehenden Ansprüche, wobei die Steuereinheit (6) geeignet ist, mit mindestens einer externen Peripherieeinheit zum Senden und/oder Empfangen mehrerer Betriebsparameter verbunden zu werden.

12. Sterilisationsverfahren, umfassend die Schritte:
- Bereitstellen des Nutzvolumens;
- Bereitstellen eines Verdampfers, der ein vakuumdichtes Auffangvolumen definiert und ein Heizelement umfasst;
- Bereitstellen eines Drucksensors;
- Bereitstellen eines Temperatursensors;
- Erfassen mittels des Drucksensors mindestens einer Messung, die sich auf den Druck innerhalb des Auffangvolumens oder innerhalb des Nutzvolumens bezieht;
- Erfassen mindestens einer Messung, die sich auf die Temperatur des Heizelements bezieht, durch den Temperatursensor;
- Zuführen einer Menge von flüssigem Wasserstoffperoxid zu dem Heizelement;
- Betreiben des Heizelements, um einen Phasenwechsel im Wasserstoffperoxid von der flüssigen Phase in die gasförmige Phase einzuleiten;
- Einleiten des Wasserstoffperoxids in der gasförmigen Phase in das Nutzvolumen;
wobei der Schritt des Zuführens des flüssigen Wasserstoffperoxids und der Schritt des Betreibens des Heizelements auf der Grundlage einer vorgegebenen Logik und auf der Grundlage der Messung, die sich auf den Druck innerhalb des Auffangvolumens oder innerhalb des Nutzvolumens bezieht, und auf der Grundlage der Messung, die sich auf die Temperatur des Heizelements bezieht, gesteuert werden.

13. Verfahren nach dem vorhergehenden Anspruch, wobei der Schritt des Erfassens mindestens einer Messung, die sich auf die Temperatur des Heizelements bezieht, durch den Temperatursensor, den Unterschritt des Erfassens mindestens einer Messung umfasst, die sich auf die Temperatur der Wärmeaustauschfläche des Heizelements bezieht.

14. Verfahren nach Anspruch 12 oder 13, ferner umfassend die Schritte:
- Bereitstellen eines Dynamometers (9);
- Erfassen durch das Dynamometer (9), um ein Signal zu erfassen, das sich auf den tatsächlichen Massenstrom des Wasserstoffperoxids bezieht;
- Vergleichen des Signals, das sich auf den tatsächlichen Massenstrom des Wasserstoffperoxids bezieht, mit dem Wert des theoretischen Massenstroms des Wasserstoffperoxids;
- Steuern der Pumpe (8) derart, um die Differenz zwischen dem tatsächlichen Massenstrom des Wasserstoffperoxids und dem theoretischen Massenstromwert des Wasserstoffperoxids zu minimieren.

## Revendications

1. Appareil de stérilisation (1), comprenant :
- des moyens (5) pour l'alimentation en peroxyde d'hydrogène en phase liquide ;
- un vaporisateur (2) adapté pour induire un changement de phase pour le peroxyde d'hydrogène de la phase liquide à la phase gazeuse, et définissant un volume de confinement (3) étanche au vide comprenant :
un capteur de température (13),
un élément chauffant (10), et
un conduit de sortie (4) pour l'écoulement du peroxyde d'hydrogène en phase gazeuse vers un volume de travail (L) ;
- un capteur de pression (15),
- une unité de commande (6)
dans lequel
le capteur de pression (15) est relié de manière opérationnelle à l'unité de commande (6), le capteur de pression (15) étant adapté pour générer un signal de pression représentant la pression à l'intérieur du volume de confinement (3) ou à l'intérieur du volume de travail (L) et pour envoyer le signal de pression à l'unité de commande (6) ;
le capteur de température (13) est relié de manière opérationnelle à l'unité de commande ;
les moyens (5) pour l'alimentation en peroxyde d'hydrogène sont reliés de manière opérationnelle à l'unité de commande (6) ;
l'élément chauffant (10) est relié de manière opérationnelle à l'unité de commande (6) ;
et dans lequel l'unité de commande (6) commande le fonctionnement de l'élément chauffant et des moyens (5) pour l'alimentation en peroxyde d'hydrogène sur la base des signaux envoyés par le capteur de température (13) et par le capteur de pression (15) et sur la base d'une logique prédéfinie ;
**caractérisé en ce que** le capteur de température (13) est adapté pour générer un signal de température représentant une température de l'élément chauffant (10) et pour envoyer le signal de température à l'unité de commande (6).

2. Appareil de stérilisation selon la revendication 1, dans lequel ledit élément chauffant (10) définit une surface d'échange de chaleur (10a), ledit élément chauffant (10) étant adapté pour recevoir une quantité prédéterminée de peroxyde d'hydrogène ; dans lequel ledit capteur de température (13) est configuré pour générer au moins un signal de température représentatif d'une température sur ladite surface d'échange de chaleur (10a) et pour envoyer le signal de température à l'unité de commande (6).

3. Appareil de stérilisation selon la revendication 1 ou 2, dans lequel le conduit de sortie (4) comprend des moyens de chauffage (12) configurés pour fournir une quantité prédéterminée de chaleur au peroxyde d'hydrogène en phase gazeuse.

4. Appareil de stérilisation selon la revendication 3, dans lequel le conduit de sortie (4) comprend un second capteur de température (14) adapté pour générer un second signal de température représentant la température du conduit de sortie (4) et pour envoyer le second signal de température à l'unité de commande (6) ; dans lequel les moyens de chauffage (12) sont reliés de manière opérationnelle à l'unité de commande (6) ; et dans lequel l'unité de commande (6) commande le fonctionnement des moyens de chauffage (12) sur la base des signaux envoyés par le second capteur de température (14) et sur la base d'une logique prédéfinie.

5. Appareil de stérilisation selon une ou plusieurs des revendications précédentes, comprenant en outre un ou plusieurs parmi :
- un troisième capteur de température (17) ;
- un capteur d'humidité relative (18) ;
- un capteur de concentration de peroxyde (19).

6. Appareil de stérilisation selon une ou plusieurs des revendications précédentes, dans lequel l'unité de commande (6) est adaptée pour commander des moyens de ventilation (16) configurés pour retirer un fluide du volume de travail (L) sur la base d'une logique préchargée.

7. Appareil de stérilisation selon la revendication précédente, comprenant en outre les moyens de ventilation (16).

8. Appareil de stérilisation selon une ou plusieurs des revendications précédentes, dans lequel les moyens (5) pour alimenter en peroxyde d'hydrogène comprennent un réservoir (7), adapté pour contenir une quantité de peroxyde d'hydrogène, et une pompe (8) configurée pour déplacer le peroxyde d'hydrogène du réservoir (7) au vaporisateur (2).

9. Appareil de stérilisation selon la revendication 8, comprenant en outre un dynamomètre (9) relié audit réservoir (7) pour obtenir une mesure de la quantité de peroxyde d'hydrogène contenue dans le réservoir (7) et configuré pour envoyer la mesure à l'unité de commande (6) .

10. Appareil de stérilisation selon une ou plusieurs des revendications précédentes, comprenant des moyens de soufflage (11) actifs sur le vaporisateur (2) pour introduire un flux de gaz ou de mélanges de gaz à l'intérieur du volume de confinement (3).

11. Appareil de stérilisation selon une ou plusieurs des revendications précédentes, dans lequel l'unité de commande (6) est adaptée pour être reliée à au moins une unité périphérique externe pour envoyer et/ou recevoir une pluralité de paramètres de fonctionnement.

12. Procédé de stérilisation, comprenant les étapes suivantes :
- fournir un volume de travail
- fournir un vaporisateur définissant un volume de confinement étanche au vide et comprenant un élément chauffant ;
- fournir un capteur de pression ;
- fournir un capteur de température ;
- détecter au moyen du capteur de pression au moins une mesure liée à la pression à l'intérieur du volume de confinement ou à l'intérieur du volume de travail ;
- détecter par le capteur de température au moins une mesure liée à la température de l'élément chauffant ;
- alimenter une quantité de peroxyde d'hydrogène liquide sur l'élément chauffant ;
- faire fonctionner l'élément chauffant pour induire un changement de phase dans le peroxyde d'hydrogène de la phase liquide à la phase gazeuse ;
- introduire le peroxyde d'hydrogène en phase gazeuse dans le volume de travail ;
dans lequel l'étape d'alimenter en peroxyde d'hydrogène liquide et l'étape de faire fonctionner l'élément chauffant sont contrôlées sur la base d'une logique prédéfinie et sur la base de la mesure liée à la pression à l'intérieur du volume de confinement ou à l'intérieur du volume de travail et sur la base de la mesure liée à la température de l'élément chauffant.

13. Procédé selon la revendication précédente, dans lequel l'étape de détecter au moyen du capteur de température au moins une mesure liée à la température de l'élément chauffant comprend la sous-étape de détecter au moins une mesure liée à la température de la surface d'échange de chaleur de l'élément chauffant.

14. Procédé selon la revendication 12 ou 13, comprenant de plus les étapes de :
- fournir un dynamomètre (9) ;
- détecter par le dynamomètre (9) pour détecter un signal lié au débit massique réel du peroxyde d'hydrogène ;
- comparer le signal lié au débit massique réel du peroxyde d'hydrogène avec la valeur du débit massique théorique du peroxyde d'hydrogène ;
- commander la pompe (8) de manière à minimiser la différence entre le débit massique réel du peroxyde d'hydrogène et la valeur théorique du débit massique du peroxyde d'hydrogène.
